# EUROPEAN PATENT SPECIFICATION

(11) **EP 2 303 340 B1**
(45) Date of publication and mention of the grant of the patent: **30.07.2014**
(21) Application number: 09771096.6
(22) Date of filing: 26.06.2009
(51) Int. Cl.: A61L 2/22, A61L 2/03, B05B 17/00

(54) **MICROAEROSOL-BASED DECONTAMINATION METHOD**
DEKONTAMINATIONSVERFAHREN AUF MIKROAEROSOL-BASIS
PROCÉDÉ DE DÉCONTAMINATION À BASE DE MICROAÉROSOL

(30) Priority: 26.06.2008 RU 2008125415
(43) Date of publication of application: 06.04.2011
(73) Proprietor: Battelle Memorial Institute, Richland, WA 99352 (US)
(72) Inventor: SVENTITSKYI, Evgeniyi, Saint Petersburg 197110 (RU); GLUSHENKO, Valeryi, Saint Petersburg 197110 (RU); TOLPAROV, Yuri, Saint Petersburg 197110 (RU); EGOROVA, Tatiana, Saint Petersburg 197110 (RU); CHERNYAEVA, Elena, Saint Petersburg 197110 (RU); KONTORINA, Nadezhda, Saint Petersburg 197110 (RU); ISKRITSKY, Viktor, Saint Petersburg 197110 (RU); RAININA, Evguenia, Kennewick, WA 99337 (US)
(74) Representative: Brown, Fraser Gregory James
(86) International application number: PCT/US2009/048765
(87) International publication number: WO 2009/158565

(56) References cited:
- EP-A1- 0 924 460
- US-A- 4 512 935
- US-A- 4 680 163
- US-A1- 2007 186 368

## Description

### Field of the Invention

The present invention relates to methods for decontamination and disinfection of enclosed environments, in a variety of fields including but not limited to agriculture, medicine, healthcare, transportation, food-processing, manufacturing, building, and other applications.

### BACKGROUND INFORMATION

Pathogenic bioagents can cause significant damage to humans, animals and the environments wherein they exist. A need exists for a method that provides efficient, environmentally friendly simultaneous decontamination of multiple bio-agents in enclosed environments like hospitals. While a variety of methods have been envisioned and proposed, an effective, environmentally safe technology that provides for reasonable and cost effective clean up of enclosed facilities with complex geometry does not really exist. The present invention is a method that meets these needs, while overcoming the limitations of the prior art.

The document US 4680163 A discloses a system for sterilizing plastic containers which makes use of an ultrasound generator to atomize a sterilizing fluid. The document US 4512935 A discloses an apparatus for blending a non-electrochemically activated liquid with a gas. Germicidal properties of the resulting liquid/gas stream are not disclosed. The document EP 0924460A 1 discloses a nozzle for use in the premix burners of a gas turbine system.

Additional advantages and novel features of the present invention will be set forth as follows and will be readily apparent from the descriptions and demonstrations set forth herein. Accordingly, the following descriptions of the present invention should be seen as illustrative of the invention and not as limiting in anyway.

### SUMMARY OF INVENTION

The present invention provides a system for disinfecting a contaminated enclosed environment according to claim 1. Also provided is a method for disinfection of an enclosed area according to claim 5. Electrochemically-activated solutions (EAS) typically comprise compositions produced by anodic or cathodic (unipolar) treatment of diluted aqueous solutions of mineral salts. This treatment gives rise to metastable states with unusual physicochemical properties. While in some embodiments described herein the electrochemically activated solution is a NaCl solution it is to be distinctly understood that the invention is not specifically limited thereto but may be variously alternatively configured utilizing any of a variety of other electrochemically activated solutions appropriate for use and readily ascertainable by a party of skill in the art. It is believed that the atomized EAS particles disperse in the air and form free radicals which cause damage to the various cells, spores and other target materials upon which they come into contact, These superactive free radicals (e.g. oxygen centered free radicals) with high penetrating capability form when these droplets desiccate. These superactive free radicals then initiate a free radical attack which continues in a chain reaction within a bioagent and results in cell/virus/spore death. The method of the present invention provides various advantages because EAS, and specifically microacrosols produced from EAS themselves are not as chemically harsh, as many liquids such as bleach are and thus do not cause damage to sensitive equipment and interior materials while still maintaining efficacy as anti biological agents.

The EAS and air are mixed at an EAS ratio (1-10):1 (by mass) with the droplets of <10 µm. This mixture is then atomized with a vortex ejector nozzle that subsequently separates the coarsely dispersed particles. In one embodiment of the invention an aqueous solution of sodium chloride, subjected to electrolysis in an anode chamber of an electrolysis device with a diaphragm, serves as an EAS for atomizing. In other embodiments this aqueous solution of sodium chloride is several times subjected to electrolysis in an anode chamber of an electrolysis device, to create the EAS with subsequently higher active ions concentrations. While these embodiments are described, any devices, materials or combinations that create a solution dispersion of an EAS having a generally neutral pH may be utilized. This process can be performed generally regardless of the humidity or temperature and is not limited by the facility size. While these particular configurations and parameters are described it is to be distinctly understood that the invention is not limited thereto but may be variously alternatively embodied to include any of a variety of additional features.

In one embodiment of the invention the method is performed by an aerosol generator positioned in a cylindrical container with a working solution, in which ejector nozzles are set up above the liquid surface so as to direct the generated aerosol flow by chord to the container wall. In such embodiments the aerosol generator may have a variety of features including an air- feed assembly, a deflector in the form of a horizontal cut-off plate, and between 1 to 6 ejector nozzles, which may be variously configured so as to be capable of turning in a generally horizontal. In some configurations the ejector nozzles are configured so that the projection of the aerosol torch central axis to the container wall forms at least one aerosol turnaround to an upper edge of the container wall. In addition, the ejector nozzles may comprises a nozzle chamber for mixing a liquidto be atomized with the air flow, directed tangentially to the nozzle chamber wall. Preferably, the cross-section area of the air-feed tube and that of the nozzle orifice are selected so as to provide air pressure excess of not less than 0.1 MPa within the nozzle chamber.

### BRIEF DESCRIPTION OF THE DRAWINGS

The invention will be better understood with reference to the annexed drawings wherein:
Fig. 1 is a schematic diagram of the device to produce aerosols, connected to an aerosol generator;
Fig. 2 is a schematic drawing of an aerosol generator
Fig. 3 is a schematic drawing of an ejector nozzle
Fig. 4 is a table showing the decontamination effectiveness of the microaerosolized solution against microbial cells and spores in one application of the present invention.
Fig. 5 is a table showing the difference in effectiveness of MAEAS and other aerosols against various bioagents.
Fig. 6 shows the decontamination effectiveness of the microaerosols as a function of particle size.
Fig. 7 shows the effectiveness of the present invention compared to methods utilizing another dispersal technology (Omron nebulizer).
Fig. 8 shows the decontaminating effectiveness of EAS as a function of aerosol generating parameters.
Fig. 9 shows VAG generator productivity and particle-size distribution as a function of nozzle orientation.
Fig. 10 shows the reduction of droplets >1µm inside the airtight chamber after pulse aerosol generation.
Fig. 11 is a table showing penetration capability of microaerosol droplets generated in the method of the present invention.
Fig. 12 shows the effectiveness of MAEAS particles >1 µm and <1µm against *B*. *cereas spores* adsorbed on a surface.
Fig. 13 is a table showing the effectiveness of MAEAS particles >1 µm and <1µm against airborne B. *cereus* spores.
Fig. 14 shows decontamination effectiveness of MEAS towards various bioagents adsorbed on different materials..
Fig. 15 shows decontamination effectiveness of MAEAS against viruses H1N1 and H5N5 airborne and adsorbed on a glass surface.
Fig. 16 shows decontamination effectiveness of MAEAS in the presence of Fe⁻²

### DETAILED DESCRIPTION OF THE INVENTION

The following description includes descriptions of various preferred modes of the present invention. It will be clear from this description of the invention that the invention is not limited to these illustrated embodiments but that the invention also includes a variety of modifications and embodiments thereto. Therefore the present description should be seen as illustrative and not limiting.

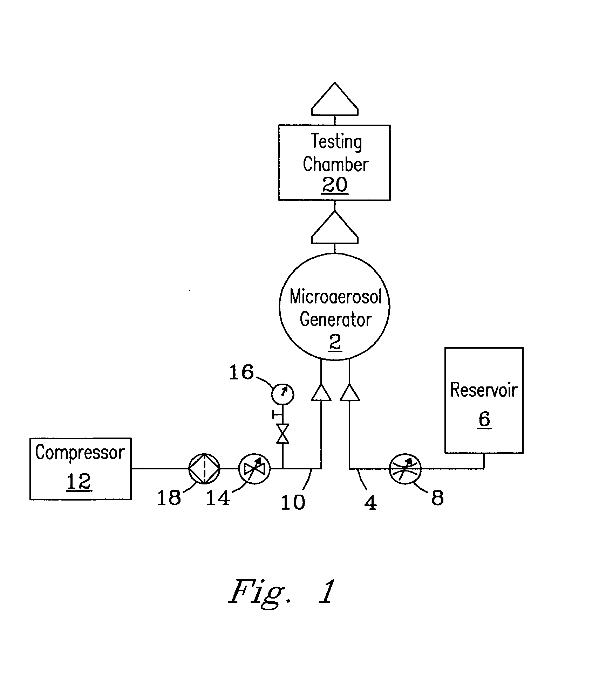

The attached figures demonstrate an example of a device that was utilized to perform the method of the present invention in various tests and applications which are set forth and described hereinafter. In these drawings, the following reference numerals refer to various features of the device that are shown in the attached Figures 1-3. The device utilized in the following experiments is made up of a microaerosol generator (2), connected to liquid-feed pipeline (4) that conducts liquid from a reservoir (6). In some embodiments a flowmeter (8) may also be included, preferably between the reservoir 6 and the aerosol generator (2). A compressed air-feed pipeline (10) connected to a motorized compressor (12) is also included. In some cases this pipeline (10) may also include a pressure controller (14) with or without a manometer (16) and/or a filter (18). In addition, the device may include a testing chamber (20) for decontamination, which is connected so as to receive a microaerosol pumped from the microaerosol generator (2).

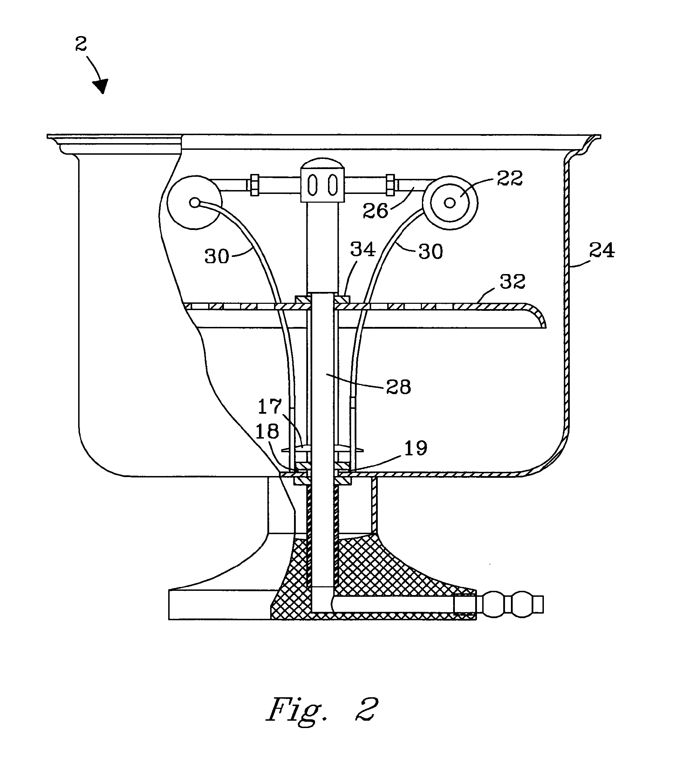

Figure 2 depicts the microaerosol generator (2) showing vortex ejection nozzles (22) positioned inside a cylindrical container (24) so that a produced microaerosol torch is directed by chord to the container wall (24). At least one nozzle (22) is required and in various alternative embodiments multiple ejection nozzles of various numbers, types and configurations of these nozzles may be provided. In this application there are preferably between 1 to 6 nozzles (22) depending upon the process area. If a particular configuration so requires, a part of one or several nozzles (22) may be replaced by plugs.

The nozzles (22) are fixed to the branch pipes (26) of a support configuration (28) that enables rotation within the container (24). The nozzles (22) are operably connected to the liquid feeding tube (4) through nozzle tubes (30), which are preferably formed from (PVC) polyvinyl chloride tubes. These tubes (30) are fixed with a ring (17), a gasket (18) and nuts (19). The structure (28) provides the nozzles (22) the ability to change positions from the top to the bottom of the container. (24). A cut-off plate (32) is affixed with a nut (34) to the support configuration (28) and enables height adjustment by moving it along the support configuration. If necessary, a diffuser may also be included on the container, which is connected detachable through a pipe with ventilation system or the testing chamber (20).

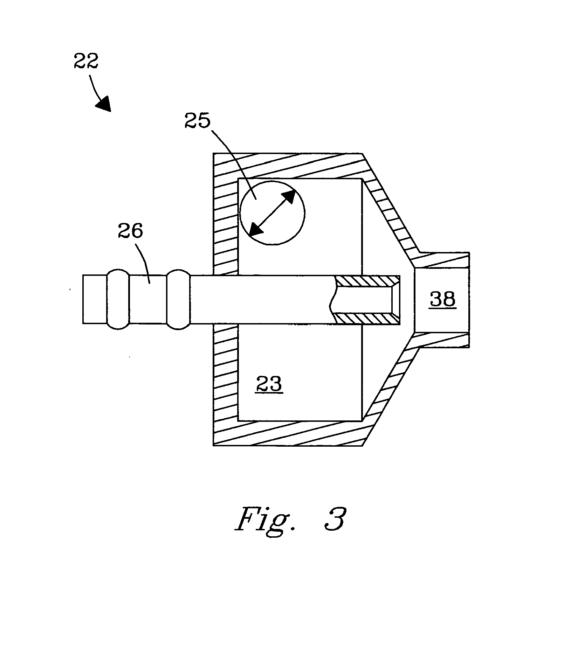

Figure 3 shows a detailed view of the ejector nozzle (22) made up of a cylindrical nozzle chamber (23) with tangential ducts (25) for air feed and the axis outlet orifice (38). A liquid-feed branch pipe (26) coaxial to the orifice (38) is set in the chamber. Our testing has shown that the highest degree of dispersion is achieved when the ratio between the cross-sectional area of the outlet orifice and the total area of the tangential duct cross-sections is 1 to 3, the length of the axis outlet orifice is 0.3-1.0 of its diameter, and the branch pipe end turned to the orifice is at the distance 0.5-2.0 of the orifice length from the exit edge of the orifice. In use the a required number of nozzles (22) are set on the branch pipes (26) of the piping lay-out (28) and appropriately spaced so as to allow sufficient coverage over the coverage area.

To apply the aerosol, working solution is fed from the reservoir (6) to the aerosol generator (2) where it is mixed with air provided from the air compressor (12). In some applications the pressure in the feed tube is set by the pressure controller (14) and can be adjusted with the manometer (16). The compressed air is fed through the filter (18) to the aerosol generator (2) wherein tangentially fed air forms the twisted flow inside the nozzle chamber (23) and then gets out through the outlet orifice (38). In these conditions, gas velocity reaches it maximum near the branch pipe (26). While along the cell axis gas is rarefied to 0.03 mPa, and the back gas flow is formed. When the air is fed from a compressor to the nozzle chamber (23) it is dehumidified to 15-20% of water content.

Liquid solution is then fed to the chamber (23) through the feed tubes (30) and the branch pipe (26) at a linear air velocity of 0.15-0.6 m/sec. The solution flow is brought by the back gas flow to the zone with maximum velocity and is broken down by centrifugal forces. The aerosol droplets thus are first time dehydrated. The generated aerosol is drawn with the air flow through the outlet orifice (38) and into the container (24). In these conditions, the air pressure decreases which causes air expansion and relative humidity reduction. Thus, the atomized liquid is further dehydrated, and the droplets reduce in size. The nozzles positioned by chord provide the two-phase flow twisting inside the container (24). As such, coarsely-dispersed droplets are settled to the container walls and plate and ran down to the bottom, while the fine-dispersed droplets are brought away from the container by the tangential air flow.

The air around the container axis is rarefied, attracting dry air flow from outside, which causes further aerosol dehydration and an increase of the concentration of droplets of about 1 µm. Thus, the concentration of particles of 1 µm increases. The produced microaerosol enters an enclosed facility or a test chamber. As the coming microaerosol is surrounded by the air "cushion" moving at the same velocity, it avoids head-on collision with room air and is not inactivated. As a result, the electro-activated microaerosol (MAEAS) preserves the activity of the liquid solution. The produced microaerosol has the higher penetrating ability as it contains a large portion of droplets of 1 µm and smaller. The following experiments demonstrated that the microaerosol of the electrochemically activated solution produced by means of the VAG generator (with the air) was ten times more effective compared to that produced with an ultrasonic generator (without air).

In one embodiment a vortex atomizer (VAG) is provided with 4 pneumatic nozzles and may operate in 3 different regimes. Operation of the atomizer in regime A (with a closed cover) results in double separation of droplets. Operation of the atomizer in regime B (with a removed cover and horizontal direction of aerosol jet) results in single separation of droplets. Operation of the atomizer in regime C (with a removed cover and the upright direction of aerosol jet) does not result in separation of coarse droplets. These regimes of operation differ in particle-size distribution in microaerosol and in productivity of the atomizer. The vortex atomizer may also be used in intermediate regimes due to change of nozzles orientation and of size of an outlet opening in the cover.

VAG's productivity and the size of aerosol droplets as a function of VAG operation regime (the mean value for 3 separate measurements)

| Regime | Productivity, ml /min | d_{g} (µm) | d_{c95} (µm) | d_{mmd} (µm) | dₘ₉₅ (µm) |
|---|---|---|---|---|---|
| A | 5±0.1 | 1.5±0.1 | 3.4±0.2 | 3.0±0.2 | 6.2±0.3 |
| B | 100±1 | 1.5±0.2 | 3.8±0.2 | 3.6±0.2 | 8.80±4 |
| C | 360±2 | 1.6±0.3 | 4.0±04 | 6.0±0.5 | 16.8±0.8 |

Where: d_{g} is counted (average geometric diameter) median diameter of the particles; d_{c95} is maximum diameter of the particles (95% of the total number of the particles); d_{mmd} is mass median diameter of the particles; and dₘ₉₅ is maximum diameter of the particles (95% of the total particles by mass).

While operating in all the regimes, the Vortex atomizer generated a fine-dispersed microaerosol (dmmd ≤ 6 µm).

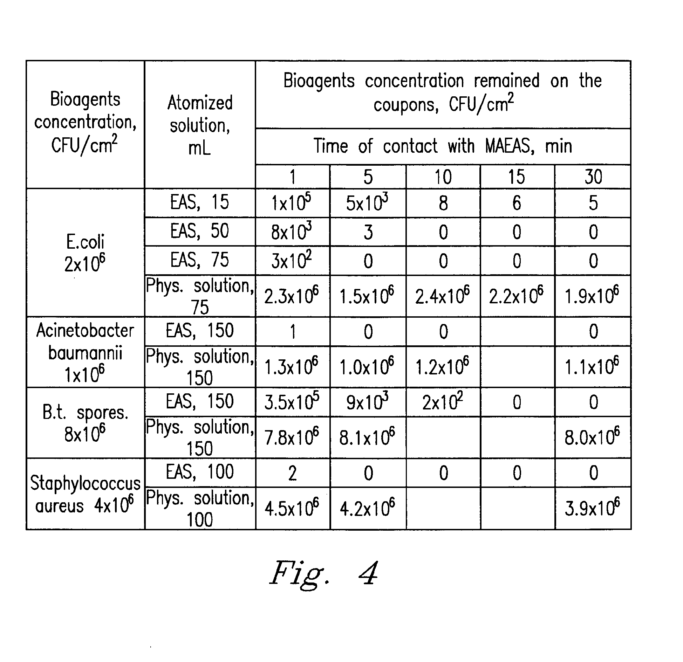

In one embodiment of the invention the decontaminating effectiveness of the droplets of this device against different microbial cells and spores applied on coupons was studied. The cell suspension was deposited on each latex-painted coupon 225 cm² in area. The contaminated coupons were dried for 1 hr. at a room temperature and positioned in the chamber of 109.3 ft³. Then 100 mL of EAS or physiological solution (control) were atomized by means of a microaerosol generator VAG to provide the aerosol droplets with dmmd =3.2 µm at the air: liquid ratio 6:1. The data obtained is shown in Figure 4. As Figure 4 shows, MAEAS demonstrated good decontamination activity against a wide spectrum of bioagents tested, including vegetative cells and spores. It also demonstrates that in some applications different bioagents require different volumes of atomized EAS and different time of a contact with MAEAS to achieve high level of decontamination.

In another embodiment of the invention, the electro-activated solution (EAS) and 1% aqueous solution of calcium hypochlorite with the same content of active chlorine - 0.1 % (by mass) were tested for efficacy against Gram-negative E. coli M17 vegetative cells, Gram- positive Staphylococcus aureus vegetative cells, and Gram- positive Bac. thuringiensis strain 98 - spores.

Glass, cotton, metal, latex paint, brick, and tile surfaces were cleaned and sterilized prior contamination. The coupon size was 225 cm². The cells and spores suspension were deposited on the coupons by means of a pneumatic atomizer generating a coarse-dispersed aerosol (droplets of 100-150 µm) to achieve 10⁶-10⁸ cells/spores/cm². Coupons with bioagents were dried at RT and at RH 50-60% for 1 hour and then were positioned inside an aerosol chamber of 109.3 ft³. Then decontaminants or physiological solutions were atomized inside the chamber at the rate 5ml/min (d_{mmd} =3.2 µm the air: liquid ratio 6:1) for a pre-determined time.

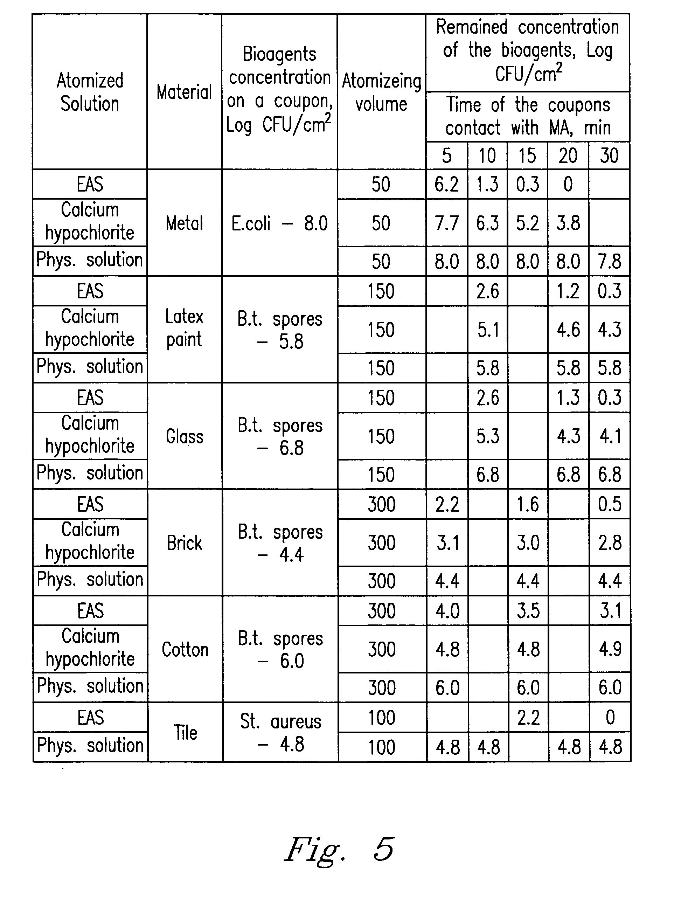

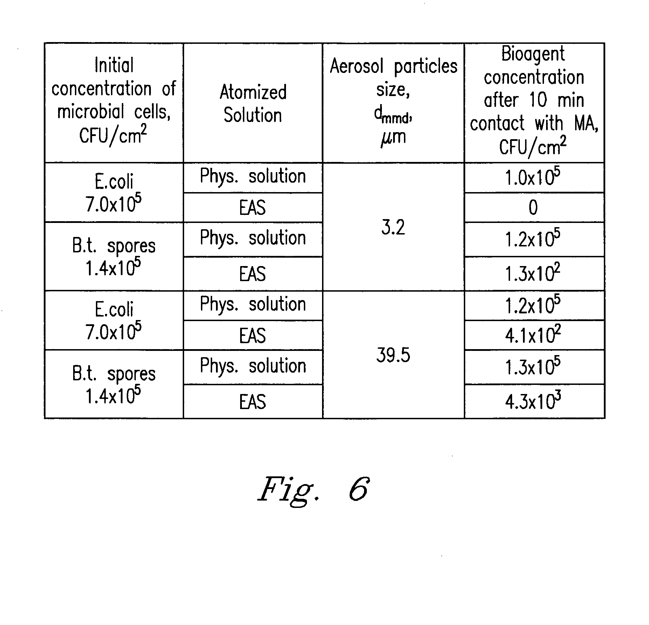

Upon experiment completion the coupons were withdrawn from the chamber and washed down with sterile physiological solution. Washed down suspensions were collected from each coupon were subjected to serial dilutions, plated on Hottinger's agar, and the colonies grown overnight were enumerated. In addition the following parameters were controlled in the course of the experiments: air sterility in a testing chamber by exposing the open Petri dishes with nutrition agar for 15 min. followed by incubation of the samples at 37±1° C for 24 hours; sterility of both physiological solution and distilled water by seeding 0.1 ml samples on nutrition agar with uniform spread of the solution with a spatula and incubating at 37±1° C for 24 hours. All experiments and controls were performed in triplicate. The results of this experiment are shown in Figures 4-6. As the results set forth in Table 5 demonstrate, the effectiveness of the method is increased as the size of the MAEAS droplet is reduced.

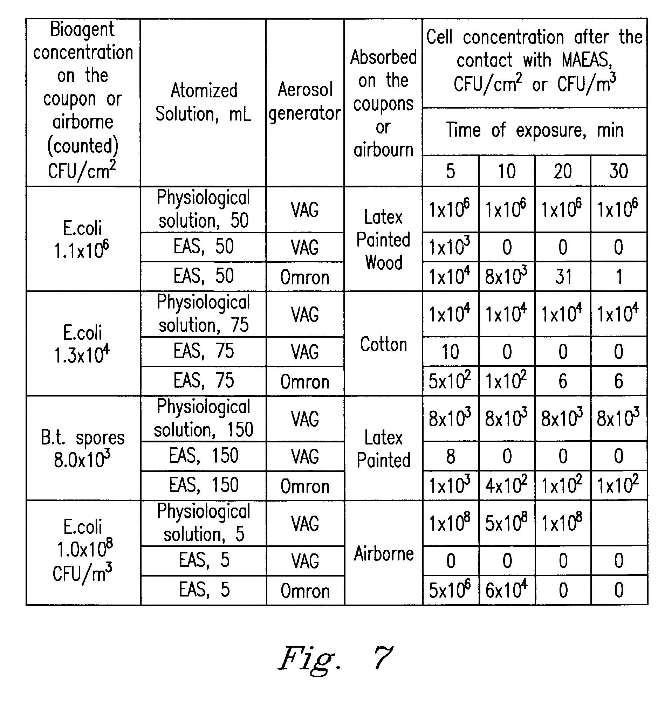

In as much as devices that produce smaller droplets have a greater efficacy in the described method. Those devices are preferred in performing the method of the present invention. However, in one set of experiments two different types of devices generating particles of mmd∼3µm were tested in the same environment to perform the method of the present invention. In these experiments, it was plainly shown that the VAG device set forth in Figures 1-3 is most effective at accomplishing the germicidal tasks that have been set forth in the present application. Figure 7 shows a comparison of the decontamination effectiveness of the MAEAS generated by VAG generator far exceeds that generated by the Omron generator (another technology). The greatest difference is seen in the short-term aerosol exposure results. This suggests a positive correlative influence of the properties of aerosol droplets produced by the VAG generator (e.g. super reactive free radicals at the droplets desiccation).

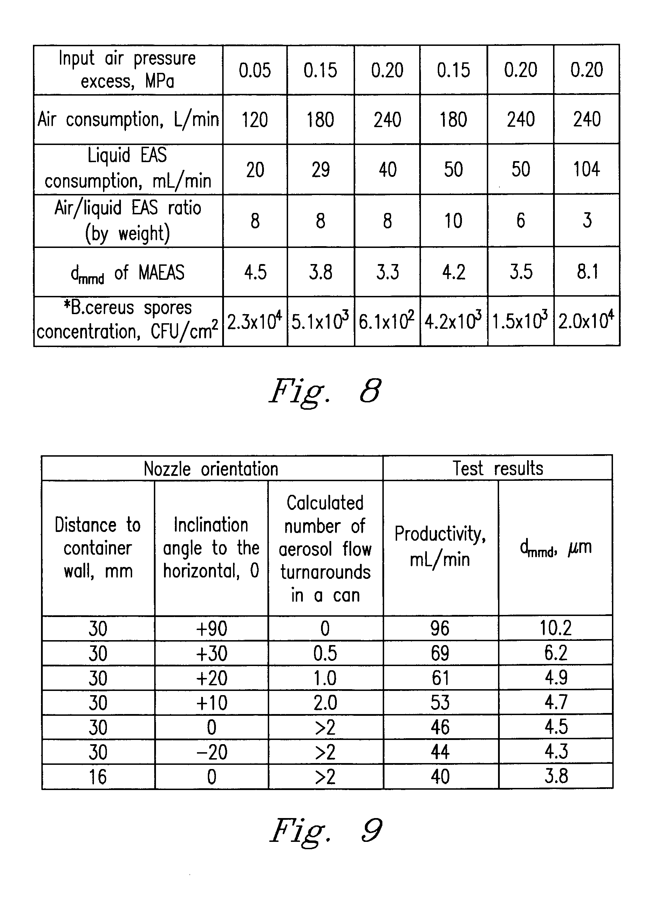

Based on the data obtained, the highest decontamination activity of MAEAS occurred when the air/liquid EAS ratio (by weight) was 8:1 and the input air pressure excess - 0.2 MPa. In addition various other factors such as the orientation of the nozzles, the length of time that the material was in the container, and other factors had an effect upon the efficacy of the decontamination method. The preferred examples are shown in the attached Figures 8-10.

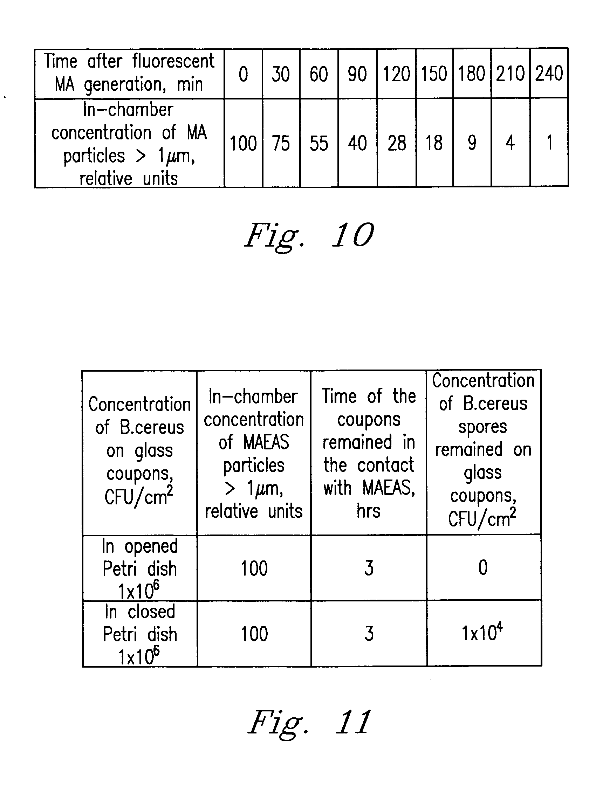

Figure 8 shows the results of decontamination effectiveness testing based upon the alteration of various characteristics of the aerosol generator. Figure 9 shows the effect of positioning and orientation of the nozzles within the container upon the production and size of particles emitted from the device. Figure 10 shows the change of the concentration (by mass) of MA droplets (mmd>1 µm) during the time of MA remains inside the chamber after the liquid was atomized. This table reflects testing wherein uranin-labeled electroactivated solution (EAS) was atomized inside the test chamber (d_{mmd} =3.6 µm). Once atomizing was terminated, air samples were periodically taken from a chamber with micro-cyclone devices and the concentration of the aerosol particles >1 µm was analyzed. The concentration of the particles immediately after EAS atomizing was nominated as 100 relative units. During 4 hours after EAS atomizing the concentration of MA droplets >1 µm decreased from 100 to one relative unit.

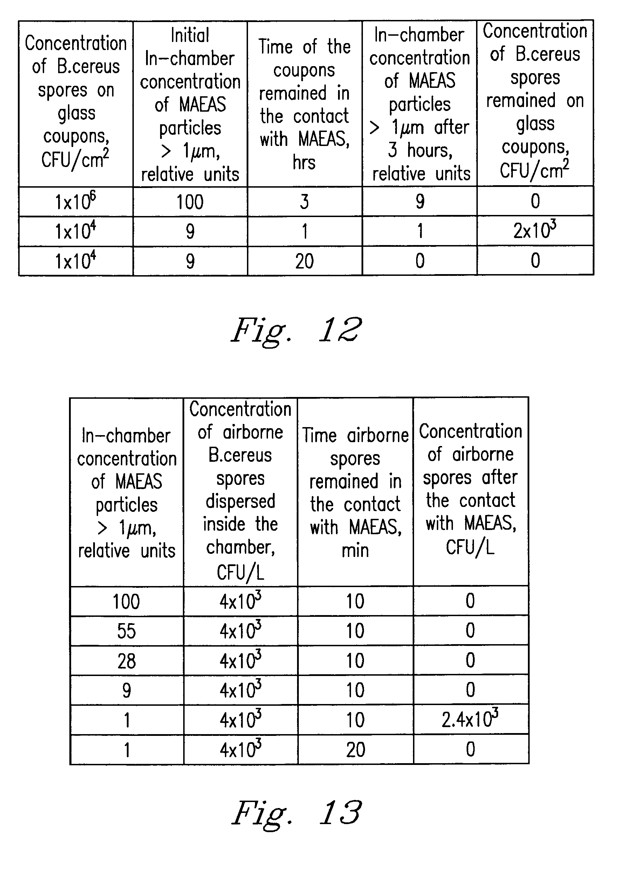

Figures 11-12 depict the data of MAEAS decontamination effectiveness after pulse aerosol generation in airtight chamber. As evident from the data, the MAEAS droplets able to penetrate the closed Petri dishes and to inactivate the spores deposited on the coupons. MAEAS droplets retain decontaminating activity, 4 hours after EAS atomizing. When the concentration of MAEAS particles > 1 µm decreased to one relative unit, there was still high decontamination effectiveness of the "chamber atmosphere". So, MAEAS preserved its decontamination activity at least during 4 hours after atomizing in contrast to the aerosols produced by analogous devices, which can preserve effectiveness for no longer than 30-40 min. This was further supported when applied to spores as described in the data found in Figure 12 and Figure 13.

As evident from the data, after deposition or desiccation of the aerosol droplets of ≥ 1 µm and larger, the "chamber atmosphere" still preserved its high bactericidal activity. As evident from the data, bactericidal activity of MAEAS remained high for at least 4 hours after atomizing, even then when almost all aerosol droplets > 1µm have deposited or desiccated. It may be speculated that this effect resulted from MAEAS droplets dehydration in the airflow and an increase of the concentration of fine-dispersed droplets in the chamber atmosphere, which possess high biocidal activity due to formation of super reactive free radicals.

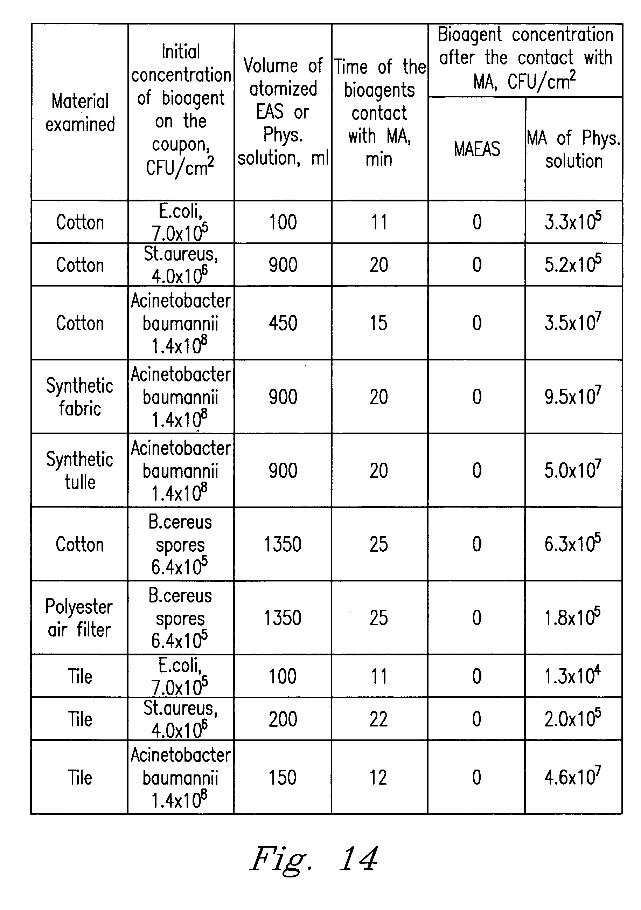

The method of the present invention was demonstrated on a variety of types of surfaces and materials and showed effective biocidal properties in each. Tables 14 and 15 demonstrate high MAEAS decontamination effectiveness against microbial cells, spores and viruses deposited on various materials. The MAEAS preserves the biocidal effectiveness for at least 4 hours and is applicable for decontamination of different materials, including fibrous cloth, conditioner filters, etc. In each of these situations and occurrences effective biocidal properties were demonstrated. See Figures 14-15.

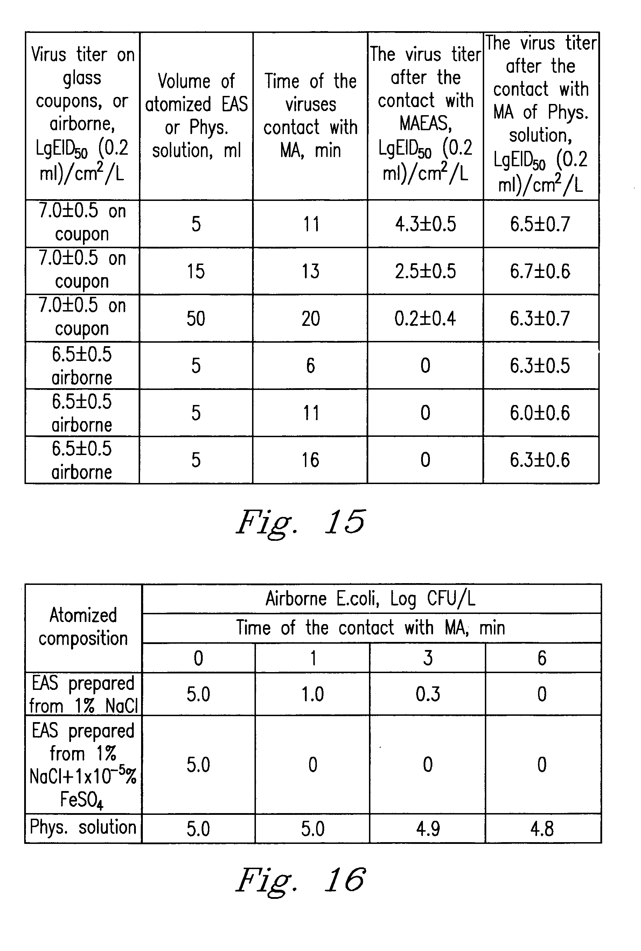

Decontaminating effectiveness of MAEAS could be increased by modification of EAS with different ions. Figure 16 demonstrates the positive effect of FeS04 added to sodium chloride for the production of MAEAS. Figure 16 shows the added effectiveness, which may be obtained by including Fe²⁺, a known free radical creating material, into the electro-activated solution. This further enhances the proposition that the present invention utilizes free-radicals as the mechanism for decontamination.

## Claims

1. A system for disinfecting an enclosed area, comprising a microaerosol which contains free radicals, said microaerosol being produced from an electrochemically-activated solution (EAS) and having droplets of ≤10 µm;
an aerosol generator (2) comprising a generally cylindrical container (24) having a circumvolving wall defining a chamber, said chamber being configured to receive a preselected quantity of a working solution therein,
said generator having at least one ejector nozzle (22) positioned within said chamber above said working solution so as to direct generated aerosol flow toward the circumvolving wall, **characterised in that**
the ejector nozzle (22) is configured so as to turn in a generally horizontal direction,
the ejector nozzle (22) is made up of a cylindrical nozzle chamber (23) with tangential ducts (25) for air feed and an outlet orifice (38); and
the ejector nozzle (22) comprises a liquid-feed branch pipe (26) coaxial to the outlet orifice (38).

2. The system of claim 1 further comprising a generally horizontally disposed deflector plate positioned within said container above said working solution.

3. The system of claim 1wherein the device comprises between 1 and 6 ejector nozzles.

4. The system of claim 1 wherein the ejector nozzle comprises a nozzle chamber for mixing a liquid to be atomized with the air flow, directed tangentially to the nozzle chamber wall.

5. A method for disinfection of an enclosed area by using a system according to claim 1, comprising injecting a microaerosol which contains free radicals into said enclosed area, said microaerosol having droplets ≤10 µm and wherein microaerosol is produced from an electrochemically-activated solution (EAS).

6. The method of claim 5 wherein the microaerosol produced from the EAS and air mixture has an air: EAS ratio of between 1-10:1 (by mass).

7. The method of claim 5 wherein the mixture is atomized by the ejector nozzle with subsequent separation of coarse dispersed particles.

8. The method of claim 5 wherein the electrochemically activated solution includes sodium chloride.

9. The method of claim 8 wherein said sodium chloride solution has a concentration of less than 5.0 g/l.

10. The method of claim 5 wherein said enclosed area has a volume greater than 5 liters.

## Patentansprüche

1. System zur Desinfektion eines umschlossenen Bereichs, umfassend ein freie Radikale enthaltendes Mikro-Aerosol, wobei das Mikro-Aerosol aus einer elektrochemisch-aktivierten Lösung (EAS) hergestellt wird und Tröpfchen von ≤ 10 µm aufweist;
einen Aerosolgenerator (2), umfassend einen im Allgemeinen zylindrischen Behälter (24) mit einer eine Kammer definierenden umgebenden Wand, wobei die Kammer so aufgebaut ist, dass sie eine vorgewählte Menge einer Arbeitslösung aufnimmt,
wobei der Generator wenigstens eine Auswurfdüse (22) aufweist, die in der Kammer über der Arbeitslösung so positioniert ist, dass ein erzeugter Aerosolfluss in Richtung der umgebenden Wand geleitet wird, **dadurch gekennzeichnet, dass** die Auswurfdüse (22) so aufgebaut ist, dass sie sich in eine im Allgemeinen horizontale Richtung dreht;
die Auswurfdüse (22) aus einer zylindrischen Düsenkammer (23) besteht, die tangentiale Leitungen (25) zur Zufuhr von Luft, und eine Auslassöffnung (38) aufweist; und die Auswurfdüse (22) ein Abzweigrohr für Flüssigkeitszufuhr (26) koaxial zu der Auslassöffnung (38) umfasst.

2. System nach Anspruch 1, ferner eine im Allgemeinen horizontal angeordnete Ablenkplatte umfassend, die innerhalb des Behälters über der Arbeitslösung positioniert ist.

3. System nach Anspruch 1, wobei die Vorrichtung zwischen 1 und 6 Auswurfdüsen umfasst.

4. System nach Anspruch 1, wobei die Auswurfdüse eine Düsenkammer zum Mischen einer zu atomisierenden Flüssigkeit mit einem Luftfluss umfasst, die tangential zu der Düsenkammerwand ausgerichtet ist.

5. Verfahren zur Desinfektion eines umschlossenen Bereichs durch Verwenden des Systems nach Anspruch 1, umfassend das Einspritzen eines freie Radikale enthaltenden Mikro-Aerosols in den umschlossenen Bereich, wobei das Mikro-Aerosol Tröpfchen von ≤ 10 µm aufweist und wobei das Mikroaerosol aus einer elektrochemisch-aktivierten Lösung (EAS) hergestellt wird.

6. Verfahren nach Anspruch 5, wobei das aus der EAS- und Luftmischung erzeugte Mikro-Aerosol ein Luft: EAS -Verhältnis zwischen 1-10:1 (nach Masse) aufweist.

7. Verfahren nach Anspruch 5, wobei die Mischung durch die Auswurfdüse mit nachfolgender Abtrennung von grob-dispersen Partikeln atomisiert wird.

8. Verfahren nach Anspruch 5, wobei die elektrochemisch aktivierte Lösung Natriumchlorid einschließt.

9. Verfahren nach Anspruch 8, wobei die Natriumchloridlösung eine Konzentration von weniger als 5,0 g/l aufweist.

10. Verfahren nach Anspruch 5, wobei der umschlossene Bereich ein Volumen von mehr als 5 Litern aufweist.

## Revendications

1. Système pour désinfecter une zone close, comprenant un microaérosol qui contient des radicaux libres, ledit microaérosol étant produit à partir d'une solution activée électrochimiquement (EAS) et comportant des gouttelettes inférieures ou égales à 10 µm ;
un générateur d'aérosol (2) comprenant un contenant (24) généralement cylindrique comportant une paroi circonférentielle définissant une chambre, ladite chambre étant configurée pour recevoir une quantité présélectionnée d'une solution de travail dans celle-ci,
ledit générateur comportant au moins une buse d'éjection (22) positionnée dans ladite chambre au-dessus de ladite solution de travail de manière à diriger le flux d'aérosol généré vers la paroi circonférentielle, **caractérisé en ce que**
la buse d'éjection (22) est configurée de manière à tourner dans une direction généralement horizontale,
la buse d'éjection (22) est constituée d'une chambre de buse (23) cylindrique avec des conduits tangentiels (25) pour une source d'air et d'un orifice de sortie (38) ; et
la buse d'éjection (22) comprend un tuyau de branchement de source de liquide (26) coaxial à l'orifice de sortie (38).

2. Système selon la revendication 1, comprenant en outre une plaque de déflecteur disposée généralement horizontalement positionnée dans ledit contenant au-dessus de ladite solution de travail.

3. Système selon la revendication 1, dans lequel le dispositif comprend entre 1 et 6 buses d'éjection.

4. Système selon la revendication 1, dans lequel la buse d'éjection comprend une chambre de buse pour mélanger un liquide à atomiser avec le flux d'air, dirigé tangentiellement à la paroi de chambre de buse.

5. Procédé pour désinfecter une zone close en utilisant un système selon la revendication 1, comprenant l'injection d'un microaérosol qui contient des radicaux libres dans ladite zone close, ledit microaérosol comportant des gouttelettes inférieures ou égales à 10 µm, et dans lequel le microaérosol est produit à partir d'une solution activée électrochimiquement (EAS).

6. Procédé selon la revendication 5, dans lequel le microaérosol produit à partir du mélange d'EAS et d'air a un rapport air: EAS entre 1:1 et 10:1 (en masse).

7. Procédé selon la revendication 5, dans lequel le mélange est atomisé par la buse d'éjection avec une séparation subséquente des grosses particules dispersées.

8. Procédé selon la revendication 5, dans lequel la solution activée électrochimiquement comprend du chlorure de sodium.

9. Procédé selon la revendication 8, dans lequel ladite solution de chlorure de sodium a une concentration inférieure à 5,0 g/l.

10. Procédé selon la revendication 5, dans lequel ladite zone close a un volume supérieur à 5 litres.
